# EUROPEAN PATENT SPECIFICATION

(11) **EP 1 165 125 B1**
(45) Date of publication and mention of the grant of the patent: **14.12.2005**
(21) Application number: 00923196.0
(22) Date of filing: 10.04.2000
(51) Int. Cl.: A61K 39/00, A61K 39/35, A61K 48/00, A61P 37/08

(54) **ANTIGEN-SPECIFIC INDUCTION OF PERIPHERAL IMMUNE TOLERANCE**
ANTIGEN-SPEZIFISCHE INDUKTION DER PERIPHEREN IMMUNTOLERANZ
INDUCTION SPECIFIQUE D'ANTIGENES DE TOLERANCE IMMUNITAIRE PERIPHERIQUE

(30) Priority: 08.04.1999 US 128420 P
(43) Date of publication of application: 02.01.2002
(73) Proprietor: The John Hopkins University, Baltimore, MD 21201 (US)
(72) Inventor: AUGUST, J., Thomas, Baltimore, MD 21210 (US); LEONG, Kam, W., Ellicot City, MD 21042 (US); GEORGANTAS, Robert, Lockport, IL 60441 (US)
(74) Representative: Tombling, Adrian George
(86) International application number: PCT/US2000/009461
(87) International publication number: WO 2000/059538

(56) References cited:
- WO-A-98/21951
- US-A- 5 633 234
- ZHANG ET AL: "INDUCTION OF SPECIFIC T CELL TOLERANCE BY FAS LIGAND-EXPRESSING ANTIGEN-PRESENTING CELLS" JOURNAL OF IMMUNOLOGY, vol. 162, 1 February 1999 (1999-02-01), pages 1423-1430, XP002151202
- SATA M ET AL: "Fas ligand gene transfer to the vessel wall inhibits neointima formation and overrides the adenovirus-mediated T cell response" PROCEEDINGS OF THE NATIONAL ACADEMY OF SCIENCES OF THE UNITED STATES,NATIONAL ACADEMY OF SCIENCE, WASHINGTON, DC,US, vol. 95, no. 3, 3 February 1998 (1998-02-03), pages 1213-1217, XP002147595 ISSN: 0027-8424
- DATABASE BIOSIS [Online] BIOSCIENCES INFORMATION SERVICE, PHILADELPHIA, PA, US; November 1997 (1997-11) DAY CHARLES S ET AL: "Myoblast-mediated gene transfer to the joint." Database accession no. PREV199800175990 XP002151203 & JOURNAL OF ORTHOPAEDIC RESEARCH, vol. 15, no. 6, November 1997 (1997-11), pages 894-903, ISSN: 0736-0266
- DATABASE BIOSIS [Online] BIOSCIENCES INFORMATION SERVICE, PHILADELPHIA, PA, US; 15 October 1998 (1998-10-15) JOSIEN REGIS ET AL: "Fas ligand, tumor necrosis factor-alpha expression, and apoptosis during allograft rejection and tolerance." Database accession no. PREV199800511546 XP002151204 & TRANSPLANTATION (BALTIMORE), vol. 66, no. 7, 15 October 1998 (1998-10-15), pages 887-893, ISSN: 0041-1337
- LEONG K W ET AL: "DNA-polycation nanospheres as non-viral gene delivery vehicles" JOURNAL OF CONTROLLED RELEASE,NL,ELSEVIER SCIENCE PUBLISHERS B.V. AMSTERDAM, vol. 53, no. 1-3, 30 April 1998 (1998-04-30), pages 183-193, XP004121269 ISSN: 0168-3659
- DATABASE BIOSIS [Online] BIOSCIENCES INFORMATION SERVICE, PHILADELPHIA, PA, US; 1995 GAO X ET AL: "Cationic liposome-mediated gene transfer." Database accession no. PREV199698648544 XP002151205 & GENE THERAPY, vol. 2, no. 10, 1995, pages 710-722, ISSN: 0969-7128
- ARAI H ET AL: "INHIBITION OF THE ALLOANTIBODY RESPONSE BY CD95 LIGAND" NATURE MEDICINE,US,NATURE PUBLISHING, CO, vol. 3, no. 8, August 1997 (1997-08), pages 843-848, XP002910396 ISSN: 1078-8956

## Description

### TECHNICAL FIELD OF THE INVENTION

This invention is related to the area of immune diseases. More particularly it relates to autoimmune diseases, allergic diseases, and transplantation rejection.

### BACKGROUND OF THE INVENTION

Immunologic tolerance to self antigens is a necessary mechanism for protecting an organism from destruction by its own immune system. When this mechanism malfunctions, allowing self-reactive immune cells to proliferate, an autoimmune disease develops within the host. A number of diseases such as Multiple Sclerosis, Lupis, Myathenia Gravis, and Rheumatoid Arthritis have been shown to result from loss of self-tolerance in T and B lymphocytes.

Fas ligand (CD95L) plays a substantial role in both the regulation [1] [2] and effector function [3] of the immune system, as well as in the maintenance of immunologic privilege [4] [5] [6-8]. T lymphocytes utilize CD95L to induce apoptosis in target cells, and also become susceptible to CD95L after antigen stimulation through the processes of Activation Induced Cell Death (AICD). [9] [10] T lymphocytes reactive with self-proteins play a substantial role in autoimmune pathology [11]. For example, Myelin reactive T cells have been demonstrated in patients with Multiple Sclerosis [12] [13] [14] and in the experimental autoimmune enchephelomyelitis mouse and rat models have been shown to be both pathogenic and able to transfer disease between syngeneic animals. [15-18]

Means for reducing an immune response to an antigen by using a combination of antigen and derivatized amino acids are known [28]. The induction of T cell tolerance to antigens expressed by antigen presenting cells through expression of fast on such cells is known [29].

There is a continuing need in the art for additional tools for treating autoimmune diseases, as well as allergic diseases and transplantation rejection.

### SUMMARY OF THE INVENTION

It is an object of the present invention to provide compositions for ablating or reducing a pathological immune response, such as an autoimmune response, an allergic response, or transplantation rejection.

It is another object of the present invention to provide compositions of nanospheres, antigen presenting cells, nucleic acid expression vectors, or liposomes useful for ablating or reducing an immune response.

These and other objects of the invention are achieved by providing compositions of nucleic acids. The nucleic acids encode a first and a second protein which are a molecule which induces apoptosis or inactivation of T cells and a pathogenic antigen, respectively. Antigen presenting cells express both the first protein and the second protein. Antigen-specific T cells are activated by the antigen presenting cells which express the pathogenic antigen. Activated antigen-specific T cells are killed or inactivated by the first protein expressed by the antigen presenting cells. Thus an immune response to the antigen can be ablated.

Also provided are nanospheres which comprise at least one nucleic acid molecule encoding a first and a second protein. The first protein induces apoptosis or inactivation of T cells, and the second protein comprises a pathogenic antigen. The pathogenic antigen is capable of activating antigen-specific T cells. The first protein is capable of killing or inactivating activated antigen-specific T cells.

Also provided by the present invention is a composition comprising a liposome which comprises at least one nucleic acid molecule encoding a first and a second protein. The first protein induces apoptosis or inactivation of T cells, and the second protein comprises a pathogenic antigen. The pathogenic antigen is capable of activating antigen-specific T cells and the first protein is capable of killing or inactivating activated antigen-specific T cells.

According to another embodiment of the invention a composition is provided which comprises a nucleic acid vector encoding a first and a second protein. The first protein induces apoptosis or inactivation of T cells, and the second protein comprises a pathogenic antigen. The pathogenic antigen is capable of activating antigen-specific T cells and the first protein is capable of killing or inactivating activated antigen-specific T cells.

In yet another embodiment of the invention a composition comprising a genetically engineered cell is provided. The cell comprises at least one nucleic acid molecule encoding a first and a second protein. The first protein induces apoptosis or inactivation ofT cells, and the second protein comprises a pathogenic antigen. The pathogenic antigen is capable of activating antigen-specific T cells and the first protein is capable of killing or inactivating activated antigen-specific T cells.

The present invention thus provides the art with compositions useful for treating autoimmune diseases, transplantation rejection, and allergic diseases.

### BRIEF DESCRIPTION OF THE DRAWINGS

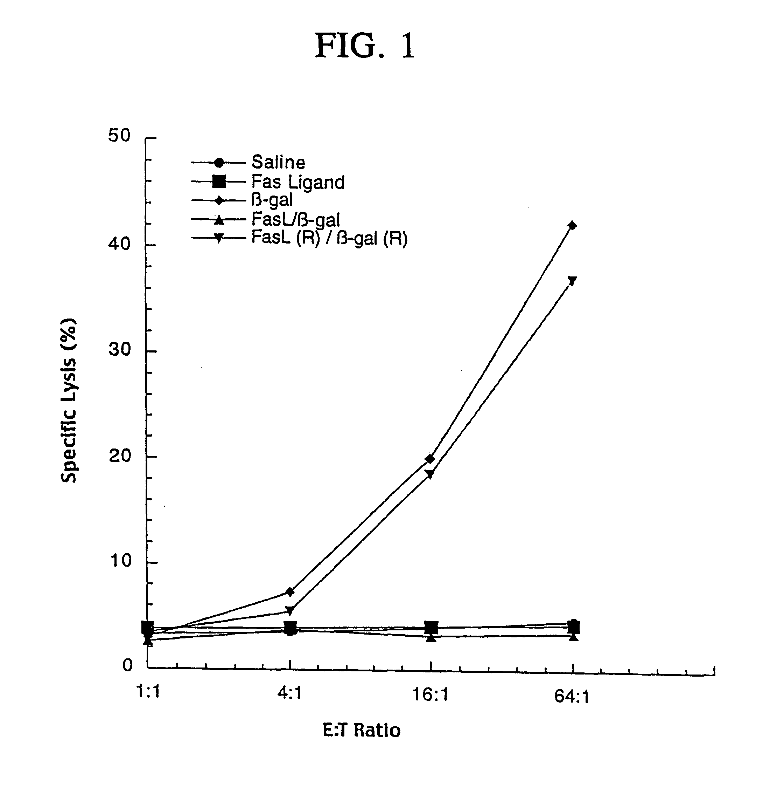

**Figure 1**. *Fas ligandILac Z co-delivery abrogates the CTL immune response to β-gal.* Mice were injected and re-injected at 2 weeks with saline alone (●), 1 *µ*g of p43-FasL DNA in nanoparticles (■), 1 *µ*g of p43-cLacZ in nanoparticles (◆), 1 *µ*g of LacZ/FasL in nanoparticles (▲), or 1 *µ*g of p43-cLacZ in nanoparticles in one leg and 1 *µ*g of p43-FasL in nanoparticles in the opposite leg (▼). T cells were isolated from the spleen and lymph nodes of the mice and assayed for β-gal specific cytotoxicity.

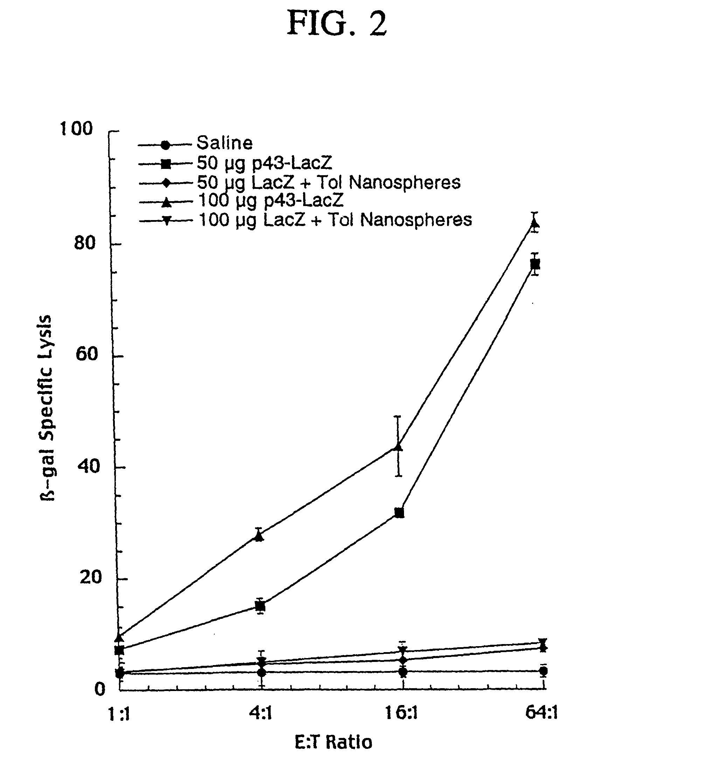

**Figure 2**. *Systemic abrogation of the* β*-gal CTL immune response by Fas ligand*/*LacZ co-delivery.* Mice were three times at two week intervals with saline alone (●), 50 µg of p43-FasL naked DNA in the left leg (■), 50 µg of p43-FasL naked DNA in the left leg and LacZ/FasL DNA nanoparticles (2 µg of DNA total) in the right leg (◆), 100 *µg* of p43-FasL naked DNA in the left leg (▲), or 100 µg of p43-FasL naked DNA in the left leg and LacZ/FasL DNA nanoparticles (2 µg of DNA total) in the right leg (▼). T cells were isolated from the spleen and lymph nodes of the mice and assayed for β-gal specific cytotoxicity.

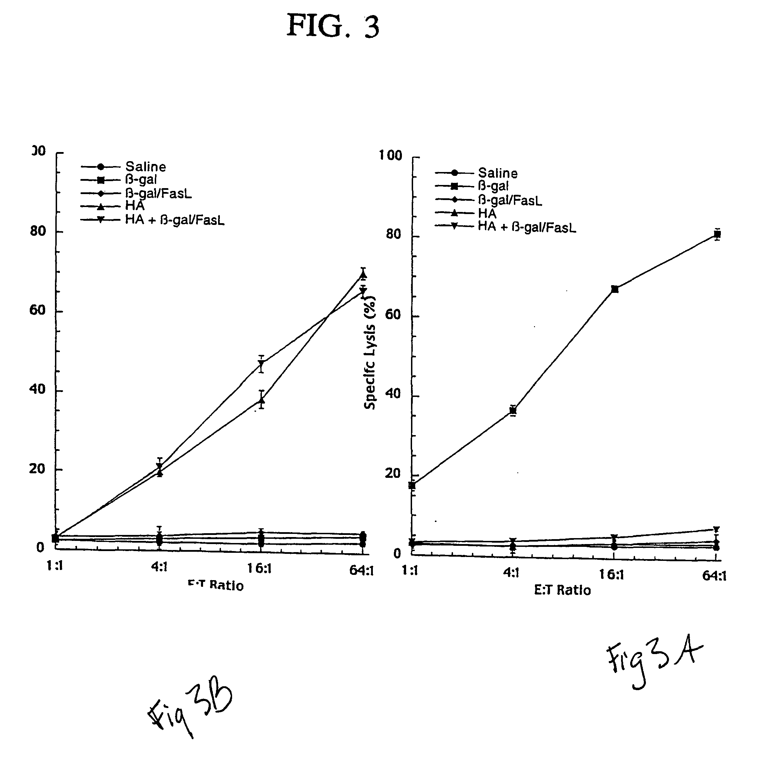

**Figure 3.** *Antigen specific induction of tolerance by LacZ*/*FasL nanoparticles.* Mice were injected at 0, 2, and 4 weeks with saline (●, p43-cLacZ nanoparticles (1 µg) (■), LacZ/FasL nanoparticles (2 µg) (◆), pcHAvector (50 *µ*g) in the left leg (▲), or p43-cLacZ nanoparticles (1 *µ*g) in the right leg and pcHA vector (50 *µ*g) in the left leg (▼). T cells harvested from the spleen and lymph nodes of and were assayed for CTL response against β-gal (Figure 3A) or to HA (Figure 3B).

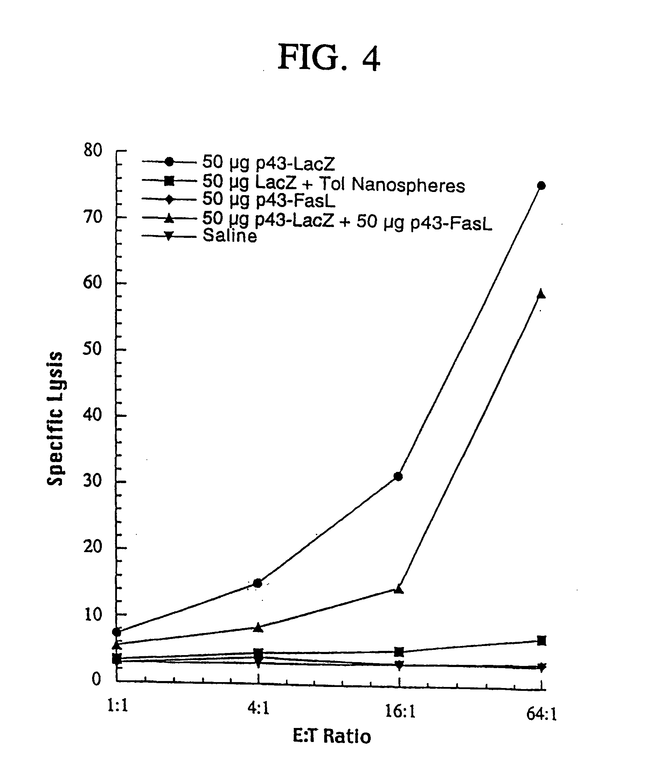

Figure 4. *Requirement for co-delivery of Fas ligand and LacZ vectors.* Mice were injected at 0, 2, and 4 weeks with saline (▼), 50 µg of naked p43-LacZ (●)**,** 50 µg of naked p43-LacZ in the left leg and LacZ/FasL DNA nanoparticles (2 µg of DNA total) in the right leg (■), 50 µg of naked p43-cLacZ (◆), a mixture of 50 µg of p43-cLacZ and *50 µg* of p43-FasL (▲). Two weeks after the final injections, T cells were harvested from the spleen and lymph nodes and were assayed for CTL response against β**-**gal.

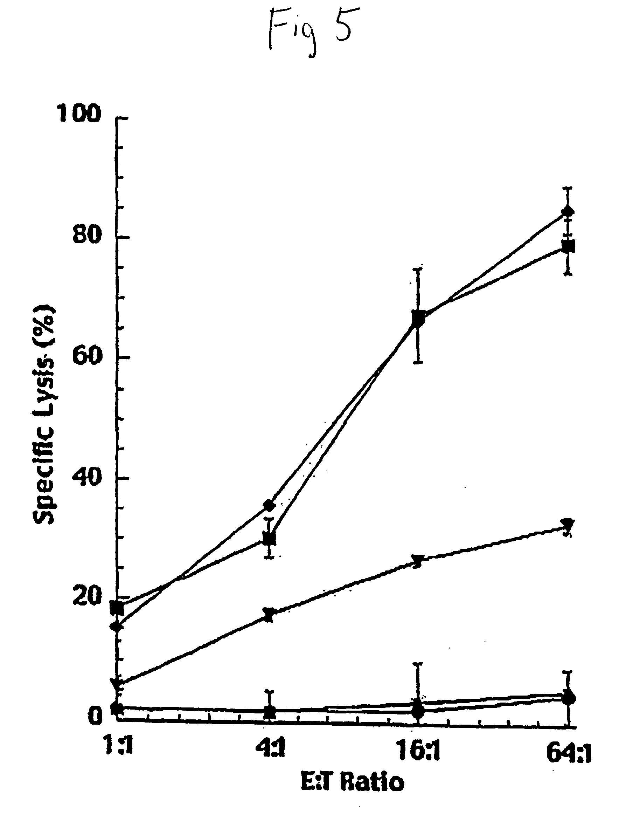

**Figure 5.** *The effects of LaZ*/*FasL nanoparticle injection on* β*-Gal immunity of pre- and post-immunized mice.* Mice were injected with saline (•), three times with 1 µg of p43-LacZ nanoparticles (■), three times with 50 µg of naked p43-LacZ (◆), twice with 2 µg of LacZ/FasL nanoparticles followed by three timer with 1 µg of p43-LacZ in nanoparticles (▲), or three times with 1 µg of p43-LacZ nano-particles followed by twice with 2 µg of LacZ/fasL nanoparticles (▼). AL1 immunization injections were given at 2-week intervals and tolerizing injections were given at 1-week intervals. Three weeks after the final injection, T cells were isolated from the spleen and lymph nodes and were assayed for β-Gal-specific cytotoxicity.

### DETAILED DESCRIPTION

It is a discovery of the present inventors that co-expression within individual cells of a pathogenic antigen and a cell protein which kills or inactivates activated T cells leads to activation and antigen-induced cell death (AICD) in reactive T cells. It is believed that interaction of T cells with protein epitopes bound in the MHC class I and II molecules of antigen presenting cells (APCs) leads to antigen-specific activation of T cells. These activated T cells then interact with a cell protein which inactivates or kills activated T cells, inducing these T cells to undergo apoptosis. It is a further finding of the inventors that nanospheres formed by coacervation of more than one coding sequence are efficient means of achieving co-transfection, without requiring special digenic constructs.

Pathogenic antigens according to the present invention are those against which a pathological immune reaction is directed. Thus the antigen can be, for example, an allergen, an autoantigen, or a transplantation antigen. Autoimmune antigens are relevant to several diseases, including Multiple Sclerosis, Rheumatoid Arthritis, Myasthenia gravis, and Lupis Erithmatosis. Other diseases in which an autoimmune component has been implicated are Insulin Dependent Diabetes Mellitus, and Crohn's disease.

Nucleic acids encoding the pathogenic antigen of choice may include a targeting signal directing the protein to the MHC class II antigen presentation system. One particularly preferrred targeting system is the LAMP-1 targeting system. See U.S. Pat. No. 5633234, which is expressly incorporated herein. The LAMP targeting system guides proteins to the endosomal/lysosomal system for processing and presentation. LAMP-mediated lysosomal targeting of antigens has been shown to increase the immune reaction to the antigen through a mechanism of increased antigen presentation to CD4+ T helper cells. In contrast, no particular targeting system is required to achieve antigen presentation in the MHC class I system. Presentation via the MHC class I system activates CD8+ cytotoxic T cells. Activation and subsquent deletion of the CD4+ and CD8+ populations abrogates both antibody and cellular immune responses.

Nucleic acids can be used which encode any cellular protein which induces apoptosis or inactivation of T cells. The cellular protein can be a surface protein or a secreted protein for which the activated T cells express a receptor. Preferably expression of the receptor is up-regulated upon activation of the T cells. Suitable cellular proteins include, without limitation, Fas ligand and TNFα. Secreted proteins such as cytokines and chemokines can be used as well.

Ablation of an immune response includes reduction, alleviation, amelioration and total elimination. The immune response can be evaluated by measuring any relevant biological component of the immune system or by evaluating symptoms of a patient or mammal being treated. See, Chapter 2, "The Induction, Measurement, and Manipulation of the Immune response," in *Immunobiology, the immune system in health and disease,* Janeway, Travers, Hunt, and Walport, Current Biology Limited, NY, 1997. Preferably a reduction of at least 25% is achieved. More preferably a reduction of at least 50%, 75%, 80%, 85%, 90%, or 95% is achieved.

Administration of nucleic acids according to the invention can be according to any procedure known in the art. While delivery of nanospheres which coacervate both coding sequences is desired, other delivery systems as are known in the art can be used, including but not limited to liposomes, viral particles, naked DNA, gene guns, and the like. Cells which have been transfected *ex vivo* can also be used for delivery of the nucleic acids to a mammal. Cells can be antigen presenting cells or muscle cells. Preferably they are autologous. Although applicants do not wish to be bound by any particular theory or mechanism of operation, it appears that co-tranfection of cells with both components, the coding sequence for the pathogenic antigen and the coding sequence for the cellular protein which induces apoptosis or inactivation, is required. Thus methods which maximize the probability of cotransfection are preferred. The two coding sequences can be on a single molecule of nucleic acid or they can be physically associated in a complex, for example. Those of skill in the art will be able to choose the precise configuration which is most convenient for the particular application at hand.

Uptake of the nucleic acids by antigen presenting cells can be accomplished as is known in the art. See Akbari et al., *J. Exp. Med. 189:*169-177; Casares et al, *J. Exp. Med.* 186:1481-1486; Condon et al, *Nature Medicine 2*:1122-1128; and Porgador et al, *J. Exp. Med. 188*:1075-1082. If a targeting moiety is desired to increase the number of antigen presenting cells which take up the DNA, one can target N418, CD83, MHC class II, CD80 and CD86 (B7.1 and B7.2) and CD40. For example, antibodies or antibody portions which are specific for these cell makers can be attached to the nanospheres.

Expression vectors which are useful according to the present invention are molecules which will ensure transcription and translation of the encoded proteins. Appropriate expression vectors are known in the art for mammalian cells and in particular for human cells. The promoters used may be constitutive or regulated. The expression units for the apoptosis-inducing protein and the pathogenic antigen may be on one or two distinct molecules.

Liposomes are well known in the art. Any technique may be used for making the liposomes at the choice of the skilled artisan. The liposomes can co-encapsulate two distinct expression vectors or one vector which encodes both the pathogenic antigen and the apoptosis-inducing protein.

Modes of administration to the mammal or patient can be by any conventional route. These include, but are not limited to subcutaneous, intramuscular, intravenous, intraperitoneal, oral, intranasal, intrabronchial, and scarification. While it is believed that the present method works by *in vivo* uptake of the DNA directly by antigen presenting cells, it is possible that other cells such as muscle cells take up the DNA as well.

Nanopsheres typically are coacervates of a positively charged and a negatively charged polymer. Nanospheres which employ nucleic acids as the negatively charged polymer can employ, for example, gelatin, chitosan, or derivatives thereof as the positively charged polymer. The nanospheres preferably are less than 5 µm and more preferably less than 3 µm. A population of nanospheres can be sized. Whereas there may be some variability in the size of the population the size of at least 50%, 75%, 85%, or 90% of the population can be applied as characterizing the population.

The above disclosure generally describes the present invention. A more complete understanding can be obtained by reference to the following specific examples which are provided herein for purposes of illustration only, and are not intended to limit the scope of the invention.

### EXAMPLE 1

### Injection of Mice with LacZ/FasL nanoparticles

Immunization of mice with gelatin nanoparticles containing a β-gal encoding p43-cLacZ vector had been previously shown to produce a large antigen specific immune reaction in mice. Also, protein molecules such as cytokines, which effect the immune system, could be encapsulated and co-delivered with the DNA vector nanoparticles resulting in a shift in the immune response toward a Th1 or Th2 bias. [25]

Since our theoretical model for antigen specific deletion of T cells required that individual cells be co-transfected with both antigen-encoding and fas ligand-encoding vectors, we tested the effect on the immune system of injecting mice with LacZ/FasL nanoparticles as compared to LacZ only nanoparticles.

We found that a normal immune response to the β-gal protein is generated when mice are immunized with LacZ vector alone or with LacZ in one leg and Fas ligand in the opposite leg (Figure 1). When co-encapsulated LacZ and Fas ligand are injected, the immune reaction against β-gal is reduced to background reactivity, similar to the group injected with saline alone. Injection of Fas ligand nanoparticles alone had no effect on the immune response. Thus, the co-delivery of a Fas ligand expression vector and an antigen-encoding vector leads to abrogation of the CTL response to the antigen.

### Methods: DNA Vectors

The p43-cLacZ vector encoding the E. coli β-galactosidase gene has been previously described [26]. The Fas ligand mRNA sequence was obtained by PCR of the gene from the pCI-FasL vector, which was the generous gift of Dr. Berry Bums. Primers for PCR of the gene sequence, sense 5'--3' and antisense 5'--3', contained EcoR I linkers. The p43-FasL vector encoding the C57B/6 mouse mRNA sequence was constructed by ligation of the DNAfragment into the EcoR I restriction site of the p43 DNA vector, and the resulting construct was sequenced by the Biochemistry Core facility to assure sequence integrity. Both vectors were propagated in DH5 cells, and were purified using the Qiagen EndoFree Plasmid Kits. Purified plasmid DNA was assayed for bacterial LPS contamination.

### Materials: Mice

6-8 week old BALB/c mice were purchased from the Charles Rivers Laboratories. All protocols for animal use were approved.

### Methods: Immunization of animals

Gelatin nanoparticles were prepared as described [24, 25] and DNA loading level was quantitated by Flourometry after digestion with 2.5% trypsin to release the DNA from the gelatin. Mice were immunized by intramuscular injection in tibialis anterior muscle of the rear legs with naked DNA or gelatin nanoparticles suspended in sterile saline solution.

### Methods: Cytotoxic T cell Assay

Lymph nodes and spleens were harvested from mice and T cells isolated by non-adherence to nylon wool as previously described and were assayed for antigen specific cytotoxicity by chromium release assay as previously described [27] [25]. Chromium release was quantitated on a Packard TopCount NXT, and results expressed as Counts × min-1 (CPM)

### EXAMPLE 2

### Systemic Abrogation of T cell Activity

Although activated APCs become refractory to apoptosis and specifically to Fas ligand mediated apoptosis by down-regulation of the Fas receptor, the possibility was raised that the expression of the Fas ligand on the surface of transfected APC's was inducing auto-apoptosis of the APC, thereby killing or inactivating the APC before it was able to interact with T cells, thereby preventing it from ever inducing an immune reaction to the antigen. To rule out this possibility, mice were immunized in one leg with a high dose of naked p43-cLacZ and in the opposite leg with LacZ/FasL nanoparticles (Figure 2). Our reasoning being that if Fas ligand expression was simply causing auto-apoptosis of transfected cells, the immune response generated in the opposite leg injected with naked p43c-cLacZ would not be effected. On the other hand, expression of antigen and the Fas ligand by individual APCs and muscle cells, which would display both activating antigen and AICD-inducing fas ligand to T cells, should lead to systemic deletion of β-gal reactive T cells.

Mice injected with naked p43-cLacZ alone developed strong CTL responses to β-gal, whereas those injected with naked p43-cLacZ in one leg and LacZ/FasL nanoparticles in the opposite leg developed responses only slightly higher than that of the group injected with saline alone. Thus, the co-delivery of fas ligand- and LacZ-encoding vectors leads to systemic abrogation of the immune response by a mechanism other than inducing apoptosis in the transfected APCs.

### EXAMPLE 3

### Abrogation of T cell Activity in Mice Immunized with Antigen Before or After Tolerization.

LacZ/FasL nanoparticles systemically abrogate the β-gal specific immuneresponse when injected at the same time as an immunization, clearly effecting the development of an immune response. However, the effectiveness against pre-existing T cells or against antigen-specific precursor T cells was not known. To answer these questions, mice were immunized three times with p43-cLacZ nanoparticles before or after injection of LacZ/FasL nanoparticles.

### EXAMPLE 4

### Antigen Specific Induction of T cell Tolerance

Having demonstrated that LacZ/FasL tolerization nanoparticles ("Tol nanospheres") abrogate both the local and systemic immune reaction to β-gal, we next asked if the tolerization injection would affect the immune response to a second antigen injected at a separate site. Mice were injected in one leg with the pcHA vector and in the opposite leg with LacZ/FasL nanoparticles. While co-delivery of LacZ and Fas ligand resulted in abrogation of the β-gal specific response (Figure 3A), the immune response to influenza hemmaglutinin (HA) was not affected (Figure 3B). These data demonstrate that the deletion of T cells induced by the LacZ/FasL nanoparticles is antigen-specific, and that the phenomenon does not affect secondary antigens that are not co-expressed with the fas ligand.

### EXAMPLE 5

### Requirement of Vector Co-Delivery

Our model of the tolerization system assumed that a co-delivery system is necessary to ensure co-expression of antigen and Fas ligand within individual cells. To test if co-delivery was in fact required (Figure 4), mice were injected with naked p43-cLacZ (50 µg), naked p43-cLacZ (50 µg) and LacZ/FasL nanoparticles (2 µg), naked p43-FasL (50 µg), or a mixture of naked p43-cLacZ (50 µg) and p43-FasL (50 *µ*g)*.* Harvested T cells were assayed for CTL activity against β-gal. The saline and p43-FasL immunization groups did not show β-gal-specific lysis activity. The p43-cLacZ group developed a strong response to β-gal. The LacZ/FasL (Tol nanospheres) with p43-cLacZ nanoparticles resulted in the abrogation of the response. The group injected with naked LacZ and FasL vectors showed only a small decrease in the immune response compared to the decrease observed in the nanoparticle group. Therefore, although a small decrease in the immune response was observed after injection of the naked vectors, co-delivery is a requirement for the abrogation of the immune response to an antigen.

In this study we have attempted to induce peripheral tolerance of T lymphocytes by taking advantage of the phenomena of Activation Induced Cell Death, in which activated T cells become susceptible to induction of apoptosis by the fas ligand. An immune response to an antigen is initiated when an APC expressing antigen in surface bound MHC class molecules interacts with a CD4+ T helper lymphocytes. The CD4+ cell becomes activated and at the same time displays the CD40 ligand (CD 154), which fully activates the APC. The APC is then able to activate CD8+ T cytotoxic lymphocytes through display of antigen on surface-bound MHC class I molecules. We speculated that co-expressing antigen and fas ligand on individual cells might cause antigen-specific activation of T cells and induction of AICD through interaction with CD95L.

We co-encapsulated two separate DNA vectors, one encoding the CD95L and one the β-galactosidase model antigen, within gelatin nanoparticles. Gelatin nanoparticles are a coacervate of DNA and gelatin that have previously been demonstrated to have utility in vaccine delivery and in gene therapy applications. See U.S. Serial Nos. 08/657,913, filed June 7, 1996, 60/071,679, filed January 16, 1998; and 60/071,746, filed January 16, 1998, which are expressly incorporated herein. Our theory of CD95L induced tolerance required that both the antigen and CD95L genes be co-expressed in individual cells. We tested nanoparticles for the ability to to deliver two distinct nucleic acid constructs to the same cell without the need of building elaborate digeneic DNA constructs.

We have shown that T cells reactive to a specific antigen are deleted in mice injected with nanoparticles encoding both the specific antigen and a surface molecule which induces apoptosis.

### EXAMPLE 6

### Multiple Sclerosis Antigens: Myelin Basic Protein

An excellent MS model exists in the SJL/J strain of mice which mirrors the human disease in both its pathology and immunological characteristics. This disease model, called experimental allergic encephalomyelitis (EAE), is used to characterize the tolerization technique for prevention and treatment of multiple sclerosis.

The myelin basic protein mRNA sequence from the SJL/J mouse was cloned by RT-PCR into a mammalian expression vector. It was subsequently subcloned into a tPA-TmC chimera mammalian expression vector. Expression of myelin basic protein with the fas ligand is used to delete mouse T cells reactive with myelin basic protein, as described above for β-galactosidase.

### EXAMPLE 7

### Inhibition of CTL activity of pre- or postimmunized mice

LacZ/FasL DNA nanoparticles systemically inhibited the β-Gal-specific CTL response when injected into mice at the same time as administration of the immunizing DNA. We have also tested the effectiveness of the tolerization method in mice with T cells already present from a previous antigen challenge or against rechallenge with antigen after the tolerization injection (Fig. 5). Mice were injected twice with LacZ/FasL. DNA nanoparticles (2 µg of DNA) either 2 weeks before or 2 weeks after they were immunized three times with 1 µg of p43-cLacZ nanoparticles. There was no significant CTL response in mice injected twice with LacZ/FasL nanoparticles before immunization with nanoparticles containing p43-cLacZ DNA alone, yielding T cell reactivity similar to that of mice injected with saline alone. Mice immunized three times with p43-cLacZ nanoparticles and then injected with LacZ/FasL nanoparticles 7 and 14 days after the last immunization showed a great decrease in, but not complete abrogation of, T cell reactivity compared with mice injected with 1 µg of p43-cLacZ nanoparticles or with 50 µg of naked p43-cLacZ. Within the time frame of this experiment, injection of the LacZ/FasL nanoparticles prevented the development of a β-Gal-specific T cell response to antigen challenge after the mice had been tolerized. Also, LacZ/fasL nanoparticles were able to markedly reduce, but not completely abrogate, the T cell reactivity to antigen generated by immunization before tolerization injections were administered.

### References

1. Van Parijs, L.A., A.K., Homeostasis and self-tolerance in the immune system: turning lymphocytes off. Science, 1998. 280(5361): p. 243-248.
2. Wang, J.L., M. J., Molecules involved in cell death and peripheral tolerance. Current Opinion in Immunology, 1997. 9(6): p. 818-825.
3. Podack, E.R., Functional significance of two cytotoxic pathways of cytotxic T lymphocytes. Journal of Leukocyte Biology, 1995. 57: p. 548-552.
4. Greil, R.E., A. Villunger, A., On The Role And Significance Of Fas (Apo-1/CD95) Ligand (FasL) Expression In Immune Privileged Tissues And Cancer Cells Using Multiple Myeloma As A Model. Leukemia & Lymphoma, 1998.
   31(5-6): p. 477.
5. Green, D.R.W., Carl F., Fas-ligand: Privilege and peril. Proceedings of the National Academy of Sciences, 1997. 94: p. 5989-5990.
6. Griffith, T.S.B., T. Fletcher, S.M. Green, D.R. Ferguson, T.A, Fas ligand-induced apoptosis as a mechanism of immune privilege. Science, 1995. 270(5239): p. 1189-1192.
7. Griffith, T.S.F., Thomas A., The role of FasL-induced apoptosis in immune privilege. Immunology Today, 1997. 18(5): p. 240-244.
8. Griffith, T.S.Y., X. Herndon, J.M. Green, D.R. Ferguson, T.A., CD95-induced apoptosis of lymphocytes in an immune privileged site induces immunological tolerance. Immunity, 1996. 5(1): p. 7-16.
9. Green, D.R.S., David W., Activation-induced apoptosis in lymphocytes. Current opinion in Immunology, 1994. 6: p. 476-487.
10. Wong, B.A., Joseph Choi, Yongwon, T cell receptor signals enhance susceptibility to Fas-mediated apoptosis. journal of experimental medicine, 1997. 186(11): p. 1939.
11. Bach, J.F.K., S. Vanendert, P.M., Are there unique autoantigens triggering autoimmune diseases. Immunological Reviews, 1998. 164: p. 139-155.
12. Vrethem, M.D., C. Ekerfelt, C. Forsberg, P. Danielsson, O. Ernerudh, J., CD4 and CD8 lymphocyte subsets in cerebrospinal fluid and peripheral blood from patients with multiple sclerosis, meningitis and normal controls. Acta Neurologica Scandinavica, 1998. 97(4): p. 215-220.
13. de Rosbo, N.K.H., Michael Ben-Nun, Avraham, Predominance of the autoimmune response to myelin oligodendrocyte glycoprotein (MOG) in multiple
   sclerosis: reactivity to the extracellula domain of MOG is directed against three main regions. European journal of immunology, 1997.27(11): p. 3059.
14. Boiko, A.N.F., O.O., Multiple sclerosis - molecular and cell mechanisms. Molecular Biology, 1995. 29(4): p. 413-426.
15. Patterson, P.Y., Transfer of allergic encephalitis in rates by means of lymph node cells. Journal of experimental medicine, 1960. 111: p. 119-133.
16. Trotter, J., et al., Characterization f T cell lines and clones from SJL/J and (BALB/cxSJL/J)F1 mice specific for myelin basic protein. Journal of Immuology, 1985. 134: p. 2322-2327.
17. Ben-Nun, A., H. Wererle, and I.R. Cohen, The rapid isolation of clonable antigen-specific lymphocyte lines cabable of mediateing autoimmune encephalomyelitis. European Journal of Immunology, 1981. 11: p. 195-199.
18. Lyons, J.-A., M.-L. Zhao, and R.B. Fritz, Pathogenesis of acute passive murine enchephalomyelitis 1. Importance of host-derived cells as determines by kinetic analysis. Journal of neuroimmunology, 1998. 86: p. 92-103.
19. Maecker, H.U., DT. DeKruyff, RH. Levy, S., Cytotoxic T cell responses to
   DNA vaccination: Dependence on antigen presentation via class II MHC. Journal of Immunology, 1998. 161(12): p. 6532-6536.
20. Akbari, O.P., N. Garcia, S. Tascon, R. Lowrie, D. Stockinger, B., DNA vaccination: Transfection and activation of dendritic cells as key events for immunity. Journal of Experimental Medicine, 1999. 189(1): p. 169-177.
21. Chattergoon, M.A.R., Tara M. Weiner, David B., Specific Immune Induction
   Following DNA-Based Immunization Through In Vivo Transfection and Activation
   of Macrophages/Antigen-Presenting Cells. Journal of immunology, 1998. 160(12): p. 5707.
22. Porgador, A.I., KR Iwasaki, A Barber, BH Restifo, NP Germain, RN., Predominant Role For Directly Transfected Dendritic Cells In Antigen Presentation To CD8(+)T Cells After Gene Gun Immunization. Journal of Experimental Medicine, 1998. 188(6): p. 1075-1082.
23. Donnelly, J.J., et al., DNA vaccines. Annual Review of Immunology, 1997.
   15: p. 617-48.
24. Truong-Le, V.L.W., Scott M. Schweibert, Erik Mao, Hai-Quan Guggino, William B. August, J. Thomas Leong, Kam W., Gene transfer by DNA-gelatin nanospheres. Archives of Biochemistry and Biophysics, 1999. 361(1): p. 47-56.
25. Truong-Le, V.L., et al., DNA-Gelatin nanoarticles as vaccines:
   immunologic effects of co-delivered cytokine proteins and protection against ebola virus, in preperation,.
26. Troung, V.L., in Pharmacology and Molecular Science. 1998, Maryland: Baltimore.
27. Coligan, J.E., in Current Protocols in Immunology. 1994.
28. WO 98/21951
29. Journal of Immunology, vol. 162, 1999, 1423-1430.

## Claims

1. Use of nucleic acids that encode a first and a second protein, wherein the first protein induces apoptosis or inactivation of T cells, and the second protein is a pathogenic antigen, in the manufacture of a composition for transfecting cells of a mammal, for reducing an immune response to the antigen in the mammal.

2. The use according to claim 1, wherein the composition is for transfection of antigen presenting cells in the mammal, thereby causing the antigen presenting cells to express the first protein and the second protein, and thereby causing antigen-specific T cells to be activated and killed.

3. The use of claim 1 wherein the composition for administration to the mammal comprises antigen presenting cells of the mammal transfected with the nucleic acids.

4. The use of claim 1 wherein the composition for administration to the mammal comprises muscle cells of the mammal transfected with nucleic acids.

5. The use of claim 1 wherein the first and second proteins are encoded by separate nucleic acid molecules.

6. The use of claim 1 wherein the pathogenic antigen is an allergen.

7. The use of claim 1 wherein the pathogenic antigen is an autoimmune antigen.

8. The use of claim 7 wherein the autoimmune antigen is one to which the mammal mounts an immune response.

9. The use of claim 1 wherein the pathogenic antigen is a transplantation antigen.

10. The use of claim 2 wherein the composition causes the first protein to be expressed on the surface of the antigen presenting cells.

11. The use of claim 2 wherein the composition causes the first protein to be secreted by the antigen presenting cells.

12. The use of claim 1 wherein the first protein is Fas ligand.

13. The use of claim 1 wherein the first protein is TNF-α.

14. The use of claim 1 wherein the second protein is lysosomally targeted.

15. The use of claim 1 wherein the second protein is not lysosomally targeted.

16. The use of claim 2 wherein the composition causes receptors for the first protein on T cells to be up-regulated upon activation of the T cells.

17. The use of claim 1 wherein the nucleic acid molecules are coacervated in nanospheres.

18. The use of claim 5 wherein the nucleic acid molecules are coacervated in nanospheres.

19. The use of claim 14 wherein the composition causes a CD4⁺ T helper cell population to be killed.

20. The use of claim 15 wherein the composition causes a CD8⁺cytolytic T cell population to be killed.

21. The use of claim 1 wherein the antigen is myelin basic protein.

22. A composition comprising a nanosphere which comprises at least one nucleic acid molecule encoding a first and a second protein, wherein the first protein induces apoptosis or inactivation of activated T cells, and the second protein comprises a pathogenic antigen, wherein the pathogenic antigen is capable of activating antigen-specific T cells.

23. A composition comprising a liposome which comprises at least one nucleic acid molecule encoding a first and a second protein, wherein the first protein induces apoptosis or inactivation of activated T cells, and the second protein comprises a pathogenic antigen, wherein the pathogenic antigen is capable of activating antigen-specific T cells.

24. A composition comprising a nucleic acid expression vector encoding a first and a second protein, wherein the first protein induces apoptosis or inactivation of activated T cells, and the second protein comprises a pathogenic antigen, wherein the pathogenic antigen is capable of activating antigen-specific T cells.

25. A composition comprising an antigen presenting cell transfected with at least one nucleic acid molecule encoding a first and a second protein, wherein the first protein induces apoptosis or inactivation of activated T cells, and the second protein comprises a pathogenic antigen, wherein the pathogenic antigen is capable of activating antigen-specific T cells, wherein the antigen presenting cell expresses both the first and the second proteins.

26. The composition of claim 22, 23 or 25 wherein the first and the second proteins are encoded by separate nucleic acid molecules.

27. The composition of claim 22, 23, 24 or 25 wherein the pathogenic antigen is an allergen.

28. The composition of claim 22, 23, 24 or 25 wherein the pathogenic antigen is an autoimmune antigen.

29. The composition of claim 22, 23, 24 or 25 wherein the pathogenic antigen is a transplantation antigen.

30. The composition of claim 22, 23, 24 or 25 wherein the first protein is a cell surface protein.

31. The composition of claim 22, 23, 24 or 25 wherein the first protein is Fas ligand.

32. The composition of claim 22, 23, 24 or 25 wherein the first protein is TNF-α

33. The composition of claim 22, 23, 24 or 25 wherein the second protein is lysosomally targeted.

34. The composition of claim 22, 23, 24 or 25 wherein the second protein is not lysosomally targeted.

35. The composition of claim 22, 23, 24 or 25 wherein receptors for the first protein on T cells are up-regulated upon activation of the T cells.

36. The composition of claim 33 wherein a CD4⁺ T helper cell population is targeted.

37. The composition of claim 34 wherein a CD8⁺ cytolytic T cell population is targeted.

38. The composition of claim 22, 23, 24 or 25 wherein the antigen is myelin basic protein.

39. The use of claim 1, wherein the composition comprises a non-viral vector containing the nucleic acids.

40. The composition of claim 25 wherein a non-viral vector transfects the antigen presenting cells with at least one nucleic acid molecule.

41. The composition of any one of claims 22 to 38 for use in therapy.

42. Use of the composition of any one of claims 22 to 38 in the manufacture of a medicament for reducing an immune response to the antigen.

## Patentansprüche

1. Verwendung von Nukleinsäuren; die für ein erstes und ein zweites Protein codieren, wobei das erste Protein Apoptose oder Inaktivierung von T-Zellen induziert, und das zweite Protein ein pathogenes Antigen ist, zur Herstellung einer Zusammensetzung zur Transfektion von Säugerzellen, zur Senkung einer Immunantwort auf das Antigen im Säuger.

2. Verwendung nach Anspruch 1, wobei die Zusammensetzung für die Transfektion der Antigen-präsentierenden Zellen im Säuger ist, dabei eine Expression des ersten Proteins und des zweiten Proteins in den Antigen-präsentierenden Zellen bewirkt und dabei eine Aktivierung und Abtötung der Antigen-spezifischen T-Zellen bewirkt.

3. Verwendung nach Anspruch 1, wobei die Zusammensetzung zur Applikation in den Säuger mit Nukleinsäuren transfizierte, Antigen-präsentierende Säugerzellen umfaßt.

4. Verwendung nach Anspruch 1, wobei die Zusammensetzung zur Applikation in den Säuger mit Nukleinsäuren transfizierte Säugermuskelzellen umfaßt.

5. Verwendung nach Anspruch 1, wobei das erste und zweite Protein von unterschiedlichen Nukleinsäuremolekülen codiert werden.

6. Verwendung nach Anspruch 1, wobei das pathogene Antigen ein Allergen ist.

7. Verwendung nach Anspruch 1, wobei das pathogene Antigen ein autoimmunes Antigen ist.

8. Verwendung nach Anspruch 7, wobei das autoimmune Antigen eines ist, gegen das der Säuger eine Immunantwort aufbaut.

9. Verwendung nach Anspruch 1, wobei das pathogene Antigen ein Transplantationsantigen ist.

10. Verwendung nach Anspruch 2, wobei die Zusammensetzung die Expression des ersten Proteins auf der Oberfläche der Antigen-präsentierenden Zellen bewirkt.

11. Verwendung nach Anspruch 2, wobei die Zusammensetzung die Sekretion des ersten Proteins von den Antigen-präsentierenden Zellen bewirkt.

12. Verwendung nach Anspruch 1, wobei das erste Protein Fas-Ligand ist.

13. Verwendung nach Anspruch 1, wobei das erste Protein TNF-α ist.

14. Verwendung nach Anspruch 1, wobei das zweite Protein ein lysosomales Target ist.

15. Verwendung nach Anspruch 1, wobei das zweite Protein kein lysosomales Target ist.

16. Verwendung nach Anspruch 2, wobei die Zusammensetzung über die Aktivierung der T-Zellen eine Hochregulierung der Rezeptoren für das erste Protein auf den T-Zellen bewirkt.

17. Verwendung nach Anspruch 1, wobei die Nukleinsäuremoleküle in Nanokügelchen koazerviert werden.

18. Verwendung nach Anspruch 5, wobei die Nukleinsäuremoleküle in Nanokügelchen koazerviert werden.

19. Verwendung nach Anspruch 14, wobei die Zusammensetzung eine Abtötung der CD4+-T-Helfer-Zellpopulation bewirkt.

20. Verwendung nach Anspruch 15, wobei die Zusammensetzung eine Abtötung der CD8+-cytolytischen T-Zellpopulation bewirkt.

21. Verwendung nach Anspruch 1, wobei das Antigen Myelinbasisprotein ist.

22. Zusammensetzung umfassend ein Nanokügelchen, das mindestens eine Nukleinsäure umfaßt, die für ein erstes und ein zweites Protein codiert, wobei das erste Protein Apoptose oder Inaktivierung aktivierter T-Zellen induziert und das zweite Protein ein pathogenes Antigen umfaßt, wobei das pathogene Antigen in der Lage ist, Antigen-spezifische T-Zellen zu aktivieren.

23. Zusammensetzung umfassend ein Liposom, das mindestens ein Nukleinsäuremolekül umfaßt, das für ein erstes und ein zweites Protein codiert, wobei das erste Protein Apoptose oder Inaktivierung aktivierter T-Zellen induziert und das zweite Protein ein pathogenes Antigen umfaßt, wobei das pathogene Antigen in der Lage ist, Antigen-spezifische T-Zellen zu aktivieren.

24. Zusammensetzung umfassend einen Nukleinsäureexpressionsvektor, der für ein erstes und ein zweites Protein codiert, wobei das erste Protein Apoptose oder Inaktivierung aktivierter T-Zellen induziert und das zweite Protein ein pathogenes Antigen umfaßt, wobei das pathogene Antigen in der Lage ist, Antigen-spezifische T-Zellen zu aktivieren.

25. Zusammensetzung umfassend eine Antigen-präsentierende Zelle, die mit mindestens einem Nukleinsäuremolekül transfiziert ist, das für ein erstes und ein zweites Protein codiert, wobei das erste Protein Apoptose oder Inaktivierung aktivierter T-Zellen induziert und das zweite Protein ein pathogenes Antigen umfaßt, wobei das pathogene Antigen in der Lage ist, Antigen-spezifische T-Zellen zu aktivieren, wobei die Antigen-präsentierende Zelle sowohl das erste als auch das zweite Protein exprimiert.

26. Zusammensetzung nach Anspruch 22, 23 oder 25, wobei das erste und das zweite Protein von unterschiedlichen Nukleinsäuremolekülen codiert werden.

27. Zusammensetzung nach Anspruch 22, 23, 24 oder 25, wobei das pathogene Antigen ein Allergen ist.

28. Zusammensetzung nach Anspruch 22, 23, 24 oder 25, wobei das pathogene Antigen ein autoimmunes Antigen ist.

29. Zusammensetzung nach Anspruch 22, 23, 24 oder 25, wobei das pathogene Antigen ein Transplantationsantigen ist.

30. Zusammensetzung nach Anspruch 22, 23, 24 oder 25, wobei das erste Protein ein Zelloberflächenprotein ist.

31. Zusammensetzung nach Anspruch 22, 23, 24 oder 25, wobei das erste Protein Fas-Ligand ist.

32. Zusammensetzung nach Anspruch 22, 23, 24 oder 25, wobei das erste Protein TNF-α ist.

33. Zusammensetzung nach Anspruch 22, 23, 24 oder 25, wobei das zweite Protein ein lysosomales Target ist.

34. Zusammensetzung nach Anspruch 22, 23, 24 oder 25, wobei das zweite Protein kein lysosomales Target ist.

35. Zusammensetzung nach Anspruch 22, 23, 24 oder 25, wobei Rezeptoren für das erste Protein auf T-Zellen über die Aktivierung von T-Zellen hochreguliert werden.

36. Zusammensetzung nach Anspruch 33, wobei das Target eine CD4+-T-Helferzellpopulation ist.

37. Zusammensetzung nach Anspruch 34, wobei das Target eine CD8+-cytolytische T-Zellpopulation ist.

38. Zusammensetzung nach Anspruch 22, 23, 24 oder 25, wobei das Antigen Myelinbasisprotein ist.

39. Verwendung nach Anspruch 1, wobei die Zusammensetzung einen Nukleinsäuren enthaltenden, nichtviralen Vektor umfaßt.

40. Zusammensetzung nach Anspruch 25, wobei ein nichtviraler Vektor die Antigen-präsentierenden Zellen mit mindestens einem Nukleinsäuremolekül transfiziert.

41. Zusammensetzung nach einem der Ansprüche 22 bis 38 zur therapeutischen Verwendung.

42. Verwendung der Zusammensetzung gemäß einem der Ansprüche 22 bis 38 zur Herstellung eines Medikaments zur Senkung einer Immunantwort auf das Antigen.

## Revendications

1. Utilisation d'acides nucléiques qui codent pour une première et une seconde protéine, dans laquelle la première protéine induit l'apoptose ou l'inactivation des lymphocytes T, et la seconde protéine est un antigène pathogène, dans la fabrication d'une composition pour transfecter les cellules d'un mammifère, pour réduire une réponse immunitaire contre l'antigène chez le mammifère.

2. Utilisation selon la revendication 1, dans laquelle la composition est pour la transfection des cellules présentant l'antigène chez le mammifère, incitant ainsi les cellules présentant l'antigène à exprimer la première protéine et la seconde protéine, et entraînant ainsi l'activation et la mort des lymphocytes T spécifiques de l'antigène.

3. Utilisation selon la revendication 1 dans laquelle la composition pour l'administration au mammifère comprend les cellules présentant l'antigène du mammifère transfecté avec les acides nucléiques.

4. Utilisation selon la revendication 1 dans laquelle la composition pour l'administration au mammifère comprend des cellules musculaires du mammifère transfecté avec des acides nucléiques.

5. Utilisation selon la revendication 1 dans laquelle les première et seconde protéines sont codées par des molécules d'acides nucléiques distinctes.

6. Utilisation selon la revendication 1 dans laquelle l'antigène pathogène est un allergène.

7. Utilisation selon la revendication 1 dans laquelle l'antigène pathogène est un antigène auto-immun.

8. Utilisation selon la revendication 7 dans laquelle l'antigène auto-immun est un antigène contre lequel le mammifère monte une réponse immunitaire.

9. Utilisation selon la revendication 1 dans laquelle l'antigène pathogène est un antigène de transplantation.

10. Utilisation selon la revendication 2 dans laquelle la composition provoque l'expression de la première protéine à la surface des cellules présentant l'antigène.

11. Utilisation selon la revendication 2 dans laquelle la composition provoque la sécrétion de la première protéine par les cellules présentant l'antigène.

12. Utilisation selon la revendication 1 dans laquelle la première protéine est un Fas-ligand.

13. Utilisation selon la revendication 1 dans laquelle la première protéine est un TNF-α.

14. Utilisation selon la revendication 1 dans laquelle la seconde protéine est ciblée de façon lysosomale.

15. Utilisation selon la revendication 1 dans laquelle la seconde protéine n'est pas ciblée de façon lysosomale.

16. Utilisation selon la revendication 2 dans laquelle la composition provoque la régulation positive des récepteurs de la première protéine qui se trouvent sur les lymphocytes T, lors de l'activation des lymphocytes T.

17. Utilisation selon la revendication 1 dans laquelle les molécules d'acides nucléique sont placées en coacervat dans les nanosphères.

18. Utilisation selon la revendication 5 dans laquelle les molécules d'acides nucléique sont placées en coacervat dans les nanosphères.

19. Utilisation selon la revendication 14 dans laquelle la composition cause la mort de la population de lymphocytes T auxiliaires CD4⁺.

20. Utilisation selon la revendication 15 dans laquelle la composition cause la mort de la population de lymphocytes T cytolytiques CD8⁺.

21. Utilisation selon la revendication 1 dans laquelle l'antigène est une protéine de base de la myéline.

22. Composition comprenant une nanosphère qui comprend au moins une molécule d'acide nucléique codant pour une première et une seconde protéine, dans laquelle la première protéine induit l'apoptose ou l'inactivation des lymphocytes T activés, et la seconde protéine comprend un antigène pathogène, où l'antigène pathogène est capable d'activer les lymphocytes T spécifiques de l'antigène.

23. Composition comprenant un liposome qui comprend au moins une molécule d'acide nucléique codant pour une première et une seconde protéine, dans laquelle la première protéine induit l'apoptose ou l'inactivation des lymphocytes T activés, et la seconde protéine comprend un antigène pathogène, où l'antigène pathogène est capable d'activer les lymphocytes T spécifiques de l'antigène.

24. Composition comprenant un vecteur d'expression d'acide nucléique codant pour une première et une seconde protéine, dans laquelle la première protéine induit l'apoptose ou l'inactivation des lymphocytes T activés, et la seconde protéine comprend un antigène pathogène, où l'antigène pathogène est capable d'activer les lymphocytes T spécifiques de l'antigène.

25. Composition comprenant une cellule présentant l'antigène avec au moins une molécule d'acide nucléique codant pour une première et une seconde protéine, dans laquelle la première protéine induit l'apoptose ou l'inactivation des lymphocytes T activés, et la seconde protéine comprend un antigène pathogène, où l'antigène pathogène est capable d'activer les lymphocytes T spécifiques de l'antigène, où la cellule présentant l'antigène exprime à la fois la première et la seconde protéines.

26. Composition selon la revendication 22, 23, 24 ou 25 dans laquelle les première et seconde protéines sont codées par des molécules d'acides nucléique distinctes.

27. Composition selon la revendication 22, 23, 24 ou 25 dans laquelle l'antigène pathogène est un allergène.

28. Composition selon la revendication 22, 23, 24 ou 25 dans laquelle l'antigène pathogène est un antigène auto-immun.

29. Composition selon la revendication 22, 23, 24 ou 25 dans laquelle l'antigène pathogène est un antigène de transplantation.

30. Composition selon la revendication 22, 23, 24 ou 25 dans laquelle la première protéine est une protéine de surface cellulaire.

31. Composition selon la revendication 22, 23, 24 ou 25 dans laquelle la première protéine est un Fas-ligand.

32. Composition selon la revendication 22, 23, 24 ou 25 dans laquelle la première protéine est un TNF-α.

33. Composition selon la revendication 22, 23, 24 ou 25 dans laquelle la seconde protéine est ciblée de façon lysosomale.

34. Composition selon la revendication 22, 23, 24 ou 25 dans laquelle la seconde protéine n'est pas ciblée de façon lysosomale.

35. Composition selon la revendication 22, 23, 24 ou 25 dans laquelle les récepteurs de la première protéine sur les lymphocytes T sont régulés positivement lors de l'activation des lymphocytes T.

36. Composition selon la revendications 33 dans laquelle une population de lymphocytes T auxiliaire CD4⁺ est ciblée.

37. Composition selon la revendication 34 dans laquelle une population de lymphocytes T cytolytiques CD8⁺ est ciblée.

38. Composition selon les revendications 22, 23, 24 ou 25 dans laquelle l'antigène est une protéine de base de la myéline.

39. Utilisation selon la revendication 1, dans laquelle la composition comprend un vecteur non-viral contenant les acides nucléiques.

40. Composition selon la revendication 25 dans laquelle un vecteur non-viral transfecte les cellules présentant l'antigène avec au moins une molécule d'acide nucléique.

41. Composition selon l'une quelconque des revendications 22 à 38 pour l'utilisation dans la thérapie.

42. Utilisation de la composition selon l'une quelconque des revendications 22 à 38 dans la fabrication d'un médicament pour réduire une réponse immunitaire contre l'antigène.
